# EUROPEAN PATENT SPECIFICATION

(11) **EP 3 824 899 B1**
(45) Date of publication and mention of the grant of the patent: **12.06.2024**
(21) Application number: 19838332.5
(22) Date of filing: 16.07.2019
(51) Int. Cl.: A61K 36/81, A61P 1/04, A61P 3/04, A61P 3/10, A61P 19/02, A61P 29/00, A61P 43/00

(54) **INFLAMMATORY CYTOKINE PRODUCTION INHIBITOR**
INHIBITOR DER PRODUKTION INFLAMMATORISCHER CYTOKINE
INHIBITEUR DE PRODUCTION DE CYTOKINE INFLAMMATOIRE

(30) Priority: 17.07.2018 JP 2018133954
(43) Date of publication of application: 26.05.2021
(73) Proprietor: Saisei Pharma Co., Ltd., Moriguchi-shi, Osaka 570-0012 (JP)
(72) Inventor: INUI, Toshio, Moriguchi-shi, Osaka 570-0011 (JP)
(74) Representative: Cabinet Beau de Loménie
(86) International application number: PCT/JP2019/027870
(87) International publication number: WO 2020/017489

(56) References cited:
- WO-A1-2007/068773
- WO-A1-2014/102546
- JP-A- 2009 538 895
- JP-A- 2012 006 889
- JP-A- 2016 196 461
- JP-A- 2016 505 037
- JP-A- 2018 501 305
- Simón Navarrete; Marcelo Alarcón; Iván Palomo: "Aqueous Extract of Tomato(Solanum lycopersicum L. ) and Ferulic Acid Reduce the Experssion of TNF~alpha and IL -1beta in LPS-Activated Macrophages", Molecules, vol. 20, no. 8, 21 August 2015 (2015-08-21), pages 15319-15329, XP055678350, DOI: 10.3390/molecules200815319
- Giuseppina Tommonaro; Annarita Poli; Rocco De Prisco; Barbara Nicolaus: "Chemical, pharmacological and biotechnological application by industrial tomato waste and analysis of antioxidative compounds in tomato hybrids", Current Topics in Biotechnology, vol. 4, 30 November 2007 (2007-11-30), pages 109-117, XP009525924, ISSN: 0972-821X
- Young-Il Kim; Shinsuke Mohri; Shizuka Hirai; Shan Lin; Tsuyoshi Goto; Chie Ohyane; Tomoya Sakamoto; Haruya Takahashi; Daisuke Shib: "Tomato extract supresses the production of proinflammatory mediators induced by interaction between adipocytes and macrophages", Bioscience , Biotechnology, and Biochemistry, vol. 79, no. 1, 2 January 2015 (2015-01-02), pages 82-87, XP055568091, ISSN: 0916-8451, DOI: 10.1080/09168451.2014.962472
- Joseph Schwager; Nathalie Richard; Bernd Mussler; Daniel Raederstorff: "Tomato Aqueous Extract Modulates the Inflammatory Profile of Immune Cells and Endothelial Cells", Molecules, vol. 21, no. 168, 29 January 2016 (2016-01-29), pages 1-15, XP055678346, DOI: 10.3390/molecules21020168
- ZHANG FANG ET AL: "Depression-like behaviors and heme oxygenase-1 are regulated by Lycopene in lipopolysaccharide-induced neuroinflammation", JOURNAL OF NEUROIMMUNOLOGY, ELSEVIER SCIENCE PUBLISHERS BV, NL, vol. 298, 6 June 2016 (2016-06-06), pages 1-8, XP029715392, ISSN: 0165-5728, DOI: 10.1016/J.JNEUROIM.2016.06.001

## Description

### TECHNICAL FIELD

The present invention relates to inflammatory cytokine production inhibitors containing a tomato extract as an active ingredient.

### BACKGROUND ART

Inflammatory cytokines are substances which are produced from lymphocytes, macrophages, and other cells and which are involved in inflammatory responses associated with bacterial or viral infection, tumors, or histological damage. For example, inflammatory cytokines such as interleukin-1 (IL-1), interleukin-6 (IL-6), and tumor necrosis factor (TNF-α) originally have purposeful functions such as activation of immune functions against invasion by pathogenic bacteria, but continuous overproduction thereof due to certain causes is known to induce a variety of diseases such as rheumatoid arthritis, ulcerative colitis, Crohn's disease, type 2 diabetes, and obesity (especially insulin resistance).

From the standpoint of neuroinflammation, they are also known to induce depression, for example.

In this context, studies have been performed for development of drugs for inhibiting the production of the inflammatory cytokines such as TNF-α in the pathological conditions mentioned above. For example, in addition to the conventional anti-inflammatory agents such as ibuprofen and indomethacin, a variety of chemical substances have been proposed (for example, see Patent Literatures 1 to 3). However, the aforementioned diseases often follow a chronic course and may require prolonged treatment. Thus, it is particularly desirable to provide a safe compound without side effects.

From the aforementioned standpoint, some studies (Patent Literatures 4 and 5) have proposed inflammatory cytokine production inhibitors which contain juice and/or extract of fruit, or the iron-binding glycoprotein lactoferrin, rather than organic compounds, as an active ingredient mainly for the purpose of ensuring safety.

An extracted component of tomatoes has an excellent effect of inhibiting the production of inflammatory cytokines. A tomato extract that significantly inhibits production of inflammatory cytokines, particularly IL-6 and TNF-α, is disclosed below.

Meanwhile, the use of a tomato extract as an anti-allergic agent has been proposed before (Patent Literature 6) .

The anti-allergic agent proposed in this patent literature is due to the effect of inhibiting release of chemical mediators such as histamine and leukotriene from mast cells according to the actual pathogenic mechanism of allergic diseases, and does not aim to inhibit the production of inflammatory cytokines as in the present invention.

From this standpoint, tomato extracts are expected to have a variety of pharmacological actions.

### CITATION LIST

### PATENT LITERATURE

Patent Literature 1: JP 2015-174850 A
Patent Literature 2: JP 2014-101329 A
Patent Literature 3: JP 2009-013106 A
Patent Literature 4: JP 2005-089304 A
Patent Literature 5: JP 2006-069995 A
Patent Literature 6: JP 2002-080387 A

Tomato extracts with cytokine production inhibitors and for preventing inflammation are shown in each of WO 2014/102546 A1, WO 2007/068773 A1, and J. Schwager et al., "Tomato Aqueous Extract Modulates the Inflammatory Profile of Immune Cells and Endothelial Cells", Molecules, vol. 21, no. 168, 29 January 2016, pages 1-15.

Young-Il Kim et al., "Tomato extract suppresses the production of proinflammatory mediators induced by interaction between adipocytes and macrophages", Bioscience, Biotechnology, and Biochemistry, vol 79, no. 1, 2 January 2015, pages 82-87, shows fractions of tomato extract with a potential to suppress inflammation by inhibiting the production of NO or proinflammatory cytokines during the interaction between adipocytes and macrophages.

G. Tommonaro et al., "Chemical, pharmacological and biotechnological application by industrial tomato waste and analysis of antioxidative compounds in tomato hybrids", Current Topics in Biotechnology, vol. 4, 30 November 2007, pages 109-117, reports a polysaccharide fraction coming from products and by-products of the tomato industry and shows that polysaccharide inhibits NF-κB activation by preventing the reactive species production and suggests for this compound a role for controlling oxidative stress and/or inflammation.

S. Navarrete et al., "Aqueous Extract of Tomato (Solanum lycopersicum L.) and Ferulic Acid Reduce the Expression of TNF-α and IL-1β in LPS-Activated macrophages", Molecules, vol. 20, no. 8, 21 August 2015, pages 15319-15329, reports for a tomato extract an inhibitory activity on the expression of TNF-α and IL-1β cytokine by inhibiting the activation of NF-κB to suggest that tomatoes and ferulic acid may contribute to prevention of chronic inflammatory diseases.

### SUMMARY OF INVENTION

### TECHNICAL PROBLEM

Accordingly, the present invention aims to provide inflammatory cytokine production inhibitors which inhibit the production of the inflammatory cytokines TNF-α and IL-6 and can be applied to drugs effective against inflammatory diseases caused by overproduction of these inflammatory cytokines, such as rheumatoid arthritis, ulcerative colitis, Crohn's disease, and type 2 diabetes.

### SOLUTION TO PROBLEM

The present invention is defined in the claims.

### ADVANTAGEOUS EFFECTS OF INVENTION

The present invention provides inflammatory cytokine production inhibitors which are highly safe via oral administration or ingestion.

The inflammatory cytokine production inhibitors provided by the present invention inhibit overproduction of inflammatory cytokines such as interleukin-1 (IL-1), interleukin-6 (IL-6), and tumor necrosis factor (TNF-α). Thus, these inhibitors serve as therapeutic agents effective against a variety of diseases caused by overproduction of these cytokines, such as rheumatoid arthritis, ulcerative colitis, and Crohn's disease, as well as type 2 diabetes, depression, and obesity. Further, these inhibitors have the advantage of providing daily healthcare effectively by daily oral ingestion.

### BRIEF DESCRIPTION OF DRAWINGS

Fig. 1 is a graph showing the results after three hours of Experimental Example 1.
Fig. 2 is a graph showing the results after six hours of Experimental Example 1.
Fig. 3 is a graph showing the results after three hours of Experimental Example 2.
Fig. 4 is a graph showing the results after six hours of Experimental Example 2.
Fig. 5 is a graph showing the results after three hours of Experimental Example 3.
Fig. 6 is a graph showing the results after six hours of Experimental Example 3.
Fig. 7 is a graph showing the results after three hours of Experimental Example 4 as a comparative example.
Fig. 8 is a graph showing the results after three hours of Experimental Example 5.
Fig. 9 is a graph showing the results after six hours of Experimental Example 5.
Fig. 10 is a graph showing the results after three hours of Experimental Example 6.
Fig. 11 is a graph showing the results after six hours of Experimental Example 6.
Fig. 12 is a graph showing the results after three hours of Experimental Example 7.
Fig. 13 is a graph showing the results after six hours of Experimental Example 7.

### DESCRIPTION OF EMBODIMENTS

As described above, the disclosed products include inflammatory cytokine production inhibitors containing a tomato extract as an active ingredient. The invention is defined in the claims.

The tomato extract, which is an active ingredient in the inflammatory cytokine production inhibitors, is an extract obtained by homogenizing ripe raw tomatoes and removing solids, i.e., obtained from squeezed juice.

The tomato material may be of any cultivar. Any cultivar eaten raw or processed that is commonly available in the market may be used.

In the preparation of squeezed juice from dried tomatoes, they are usually homogenized together with an appropriate buffer solution such as purified water or saline. Ripe raw tomatoes having high water content may be directly crushed without using a buffer solution, and solids may then be removed by a usual method such as centrifugation to obtain squeezed juice.

Then, a tomato extract is obtained from this squeezed juice. The extraction is carried out using a centrifugal ultrafilter to obtain the extract as a fraction having a molecular weight of 10,000 or higher or, since squeezed juice of raw tomatoes contains a large amount of saccharides, the extract is preferably adjusted to 10,000 or higher on a molecular weight basis.

Examples of the centrifugal ultrafilter used include a variety of filters that may be suitable for the target molecular weight fractionation, such as Amicon Ultra^{®} series available from Merck Millipore.

An extract having a molecular weight of 10,000 or higher is obtained as a raw tomato extract. The resulting extract may be used as it is. Alternatively, it may be prepared into a concentrated liquid by solvent evaporation or prepared into a dried product before use.

The drying may be carried out by a usual drying technique such as vacuum drying, freeze drying, or spray drying. Among these, freeze drying is preferably performed to form a dried product.

The raw tomato extract has an excellent effect in inhibiting the production of inflammatory cytokines such as IL-1, IL-6, and TNF-α, and is useful as an inflammatory cytokine production inhibitor.

The inflammatory cytokine production inhibitors of the present invention may be administered in any manner in accordance with the purpose of administration, the type of disease, and the particular symptoms. The inhibitors may be in the dosage form of a tablet, a capsule, granules, powder, a powdered medicine, liquid, or other forms for direct administration or may be mixed into a food or drinking water for administration, and are desirably orally administered.

These dosage forms can be prepared by usual conventional methods. Appropriate additives such as dextrin, lactose, cornstarch, emulsifiers, antiseptics, vehicles, expanders, sweetening agents, flavors, and colorants may be incorporated as long as the advantageous effect of the present invention is not impaired.

The inflammatory cytokine production inhibitors of the present invention may also be used as foods or drinks. Examples of such foods or drinks include non-alcoholic drinks such as soft drinks and juices, alcoholic drinks, fermented drinks such as yogurt, hard sweets such as tablets and candies, chewable sweets such as gum and gummies, nutritional supplements containing, for example, vitamins, minerals, amino acids, or proteins, and other forms.

Examples of such foods or drinks functionally include foods for specified health uses ("TOKUHO"), foods with nutrient function claims, and foods with function claims.

The foregoing agents and foods and drinks have an ability to inhibit the production of inflammatory cytokines and can be very useful for the prevention, treatment, improvement or relapse prevention of a variety of pathological conditions induced by overproduction of inflammatory cytokines.

For the inflammatory cytokine production inhibitors of the present invention, the dose for achieving the ability to inhibit the production of inflammatory cytokines may vary in accordance with the purpose of administration, the type of disease, and the particular symptoms. For example, the amount may be adjusted so that 1 to 5000 mg, preferably 5 to 1000 mg, more preferably 10 to 200 mg, of the raw tomato extract can be ingested per day.

The raw tomato extract is obtained from squeezed juice of ripe tomatoes which are eaten daily, and thus is highly safe and causes no toxicity problem at the doses used in the present invention.

### EXAMPLES

Aspects of the present invention are described in more detail below with reference to examples and experimental examples.

### Example 1: Preparation of raw tomato extract

An amount of 103.96 g of ripe raw tomatoes (grown in Kagoshima Prefecture) were cut and crushed in a mortar. The resulting material was centrifuged at 4000 × g for 15 minutes and 27.5 mL of the liquid portion was collected.

A 20-mL portion of the liquid portion was subjected to centrifugal ultrafiltration at 4000 × g using the centrifugal ultrafilter Amicon Ultra-15 (MWCO: 10,000).

After the centrifugation, a high-molecular-weight fraction having a molecular weight of 10,000 or higher and a low-molecular-weight fraction having a molecular weight of 10,000 or lower were taken and freeze-dried to obtain raw tomato extracts.

The raw tomato extract prepared as above mainly contains saccharides with some proteins and can be directly used as an inflammatory cytokine production inhibitor.

The raw tomato extract in the present invention corresponds to the molecular weight fraction having a molecular weight of 10,000 or higher prepared as above, but for the purpose of comparison, the prepared molecular weight fraction having a molecular weight of 10,000 or lower was also subjected to the following experiments.

In the following experiments, the prepared raw tomato extracts were each adjusted to a concentration of 100 ug/mL in a DMEN medium (Dulbecco's modified Eagle's medium + 10% bovine serum + 0.1% antibiotic/antimicrobial) before application to cells.

### Experimental Example 1: effect of raw tomato extract on TNF-α mRNA expression in mouse microglial cells (MG6) in presence of LPS

The following describes a specific procedure.
(1) A 6-well plate was seeded with a mouse microglial cell line (MG6) at 1 × 10⁵ cells/mL.
(2) After three to four days, solutions of four groups, i.e., control (DMEN medium, 1 mL/well), LPS (5 ng/mL), LPS (5 ng) + high-molecular-weight raw tomato extract (100 ug) (liquid volume: 1 mL), and high-molecular-weight raw tomato extract (100 ug/mL) were applied to the cells.
(3) After three hours and after six hours, the culture medium was removed and 0.3 mL of an RLT solution (RNeasy Mini kit, QIAGEN) (containing 1% β-mercaptoethanol) was added to each well.
(4) The cells were scraped using a cell scraper.
(5) The cell suspension was mixed with 0.3 mL of 70% ethanol.
(6) The above solution was put into a spin column (RNeasy Mini kit, QIAGEN) and centrifuged at 8000 × g for one minute.
(7) To the spin column was added 700 µL of an RW1 solution (RNeasy Mini kit, QIAGEN), followed by centrifugation at 8000 × g for one minute.
(8) To the spin column was added 500 µL of an RPE solution (RNeasy Mini kit, QIAGEN), followed by centrifugation at 8000 × g for one minute. This step was repeated twice.
(9) The spin column was placed in a new tube (1.5 mL) and 50 µL of RNase-free water was added. The mixture was left to stand for one minute, and then centrifuged at 12000 × g for one minute. This step was repeated twice.
(10) A 11-µL portion of this aqueous solution was used with Versco cDNA Synthesis Kit (Thermoscientific) to prepare cDNA.
(11) The resulting cDNA solution was diluted 10-fold with RNase-free water.
(12) This diluted cDNA solution was used to determine the expression of GAPDH mRNA and TNF-α mRNA by standard real-time RT-PCR.
(13) The PCR was performed using a 96-well plate.
(14) Each well was charged with 8.5 µL of water, 2.5 µL of cDNA sample solution, 1.5 µL of GAPDH mRNA detection primers or TNF-α mRNA detection primers (forward + reverse), and 12.5 µL of Syber green mater mix.
(15) The thermal cycle conditions for PCR were as follows: Hold, 94°C, 10 min + 3 step PCR (40 cycles; 94°C, 30 min + 55°C, 30 sec + 72°C, 30 sec) + Hold, 72°C, 1 min.
(16) For each sample of each group, the TNF-α mRNA/GAPDH mRNA value was determined and converted into percentage with the average value of the control taken as 100%.
(17) A statistical significance test was performed by analysis of variance followed by the Tukey-Kramer method.

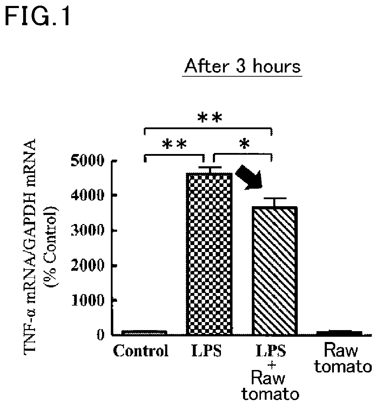

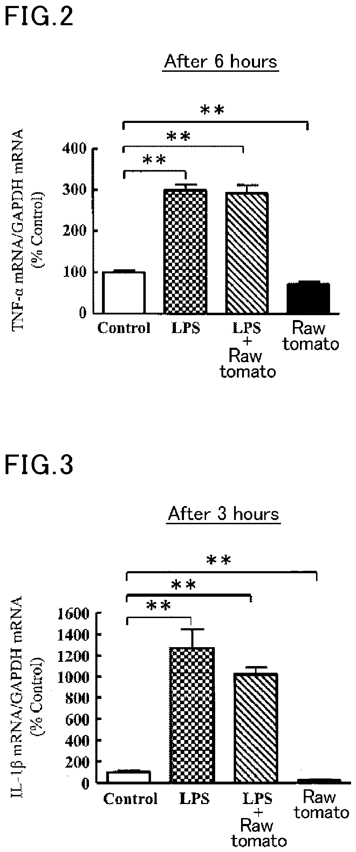

The results are shown in Figs. 1 and 2.

Fig. 1 shows the results after three hours and Fig. 2 shows the results after six hours.

As demonstrated from the results shown in Fig. 1, the raw tomato extract is understood to effectively inhibit the expression of TNF-α mRNA in the results after three hours.

### Experimental Example 2: effect of raw tomato extract on IL-1β mRNA expression in mouse microglial cells (MG6) in presence of LPS

The following describes a specific procedure, which follows Experimental Example 1.
(1) A 6-well plate was seeded with a mouse microglial cell line (MG6) at 1 × 10⁵ cells/mL.
(2) After three to four days, solutions of four groups, i.e., control (DMEN medium, 1 mL/well), LPS (5 ng/mL), LPS (5 ng) + high-molecular-weight raw tomato extract (100 ug) (liquid volume: 1 mL), and high-molecular-weight raw tomato extract (100 ug/mL) were applied to the cells.
(3) After three hours and after six hours, the culture medium was removed and 0.3 mL of an RLT solution (RNeasy Mini kit, QIAGEN) (containing 1% β-mercaptoethanol) was added to each well.
(4) The cells were scraped using a cell scraper.
(5) The cell suspension was mixed with 0.3 mL of 70% ethanol.
(6) The above solution was put into a spin column (RNeasy Mini kit, QIAGEN) and centrifuged at 8000 × g for one minute.
(7) To the spin column was added 700 µL of an RW1 solution (RNeasy Mini kit, QIAGEN), followed by centrifugation at 8000 × g for one minute.
(8) To the spin column was added 500 µL of an RPE solution (RNeasy Mini kit, QIAGEN), followed by centrifugation at 8000 × g for one minute. This step was repeated twice.
(9) The spin column was placed in a new tube (1.5 mL) and 50 µL of RNase-free water was added. The mixture was left to stand for one minute, and then centrifuged at 12000 × g for one minute. This step was repeated twice.
(10) A 11-µL portion of this aqueous solution was used with Versco cDNA Synthesis Kit (Thermoscientific) to prepare cDNA.
(11) The resulting cDNA solution was diluted 10-fold with RNase-free water.
(12) This diluted cDNA solution was used to determine the expression of GAPDH mRNA and IL-1β mRNA by standard real-time RT-PCR.
(13) The PCR was performed using a 96-well plate.
(14) Each well was charged with 8.5 µL of water, 2.5 µL of cDNA sample solution, 1.5 µL of GAPDH mRNA detection primers or IL-1β mRNA detection primers (forward + reverse), and 12.5 µL of Syber green mater mix.
(15) The thermal cycle conditions for PCR were as follows: Hold, 94°C, 10 min + 3 step PCR (40 cycles; 94°C, 30 min + 55°C, 30 sec + 72°C, 30 sec) + Hold, 72°C, 1 min.
(16) For each sample of each group, the IL-1β mRNA/GAPDH mRNA value was determined and converted into percentage with the average value of the control taken as 100%.
(17) A statistical significance test was performed by analysis of variance followed by the Tukey-Kramer method.

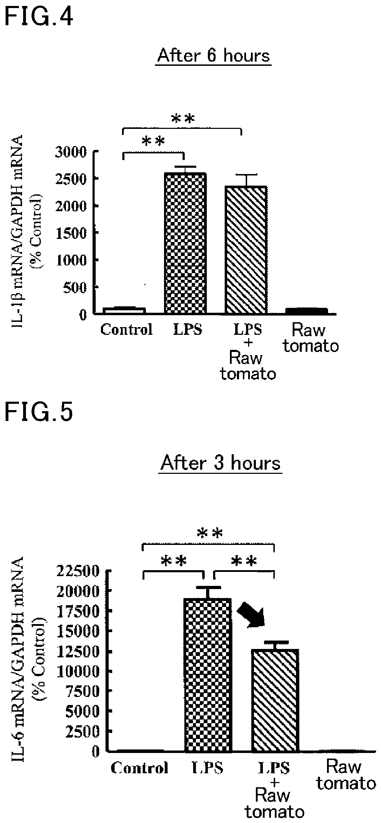

The results are shown in Figs. 3 and 4.

Fig. 3 shows the results after three hours and Fig. 4 shows the results after six hours.

As demonstrated from the results shown in the figure, the raw tomato extract is understood to effectively inhibit the expression of IL-1β mRNA in the results after three hours.

### Experimental Example 3: effect of raw tomato extract on IL-6 mRNA expression in mouse microglial cells (MG6) in presence of LPS

The following describes a specific procedure, which follows Experimental Example 1.
(1) A 6-well plate was seeded with a mouse microglial cell line (MG6) at 1 × 10⁵ cells/mL.
(2) After three to four days, solutions of four groups, i.e., control (DMEN medium, 1 mL/well), LPS (5 ng/mL), LPS (5 ng) + high-molecular-weight raw tomato extract (100 ug) (liquid volume: 1 mL), and high-molecular-weight raw tomato extract (100 ug/mL) were applied to the cells.
(3) After three hours and after six hours, the culture medium was removed and 0.3 mL of an RLT solution (RNeasy Mini kit, QIAGEN) (containing 1% β-mercaptoethanol) was added to each well.
(4) The cells were scraped using a cell scraper.
(5) The cell suspension was mixed with 0.3 mL of 70% ethanol.
(6) The above solution was put into a spin column (RNeasy Mini kit, QIAGEN) and centrifuged at 8000 × g for one minute.
(7) To the spin column was added 700 µL of an RW1 solution (RNeasy Mini kit, QIAGEN), followed by centrifugation at 8000 × g for one minute.
(8) To the spin column was added 500 µL of an RPE solution (RNeasy Mini kit, QIAGEN), followed by centrifugation at 8000 × g for one minute. This step was repeated twice.
(9) The spin column was placed in a new tube (1.5 mL) and 50 µL of RNase-free water was added. The mixture was left to stand for one minute, and then centrifuged at 12000 × g for one minute. This step was repeated twice.
(10) A 11-µL portion of this aqueous solution was used with Versco cDNA Synthesis Kit (Thermoscientific) to prepare cDNA.
(11) The resulting cDNA solution was diluted 10-fold with RNase-free water.
(12) This diluted cDNA solution was used to determine the expression of GAPDH mRNA and IL-6 mRNA by standard real-time RT-PCR.
(13) The PCR was performed using a 96-well plate.
(14) Each well was charged with 8.5 µL of water, 2.5 µL of cDNA sample solution, 1.5 µL of GAPDH mRNA detection primers or IL-6 mRNA detection primers (forward + reverse), and 12.5 µL of Syber green mater mix.
(15) The thermal cycle conditions for PCR were as follows: Hold, 94°C, 10 min + 3 step PCR (40 cycles; 94°C, 30 min + 55°C, 30 sec + 72°C, 30 sec) + Hold, 72°C, 1 min.
(16) For each sample of each group, the IL-6 mRNA/GAPDH mRNA value was determined and converted into percentage with the average value of the control taken as 100%.
(17) A statistical significance test was performed by analysis of variance followed by the Tukey-Kramer method.

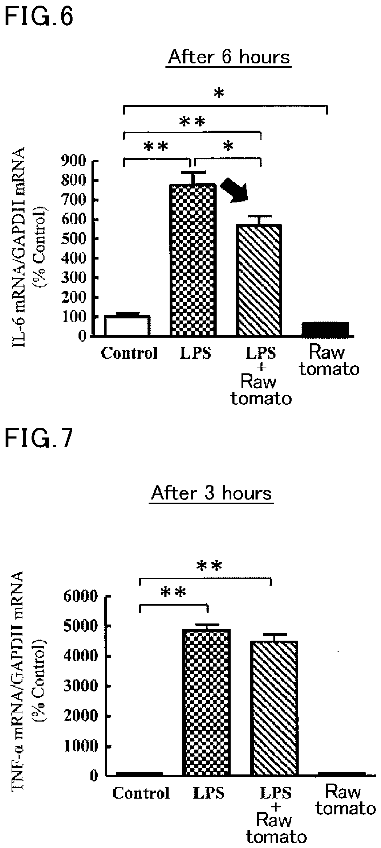

The results are shown in Figs. 5 and 6.

Fig. 5 shows the results after three hours and Fig. 6 shows the results after six hours.

As demonstrated from the results shown in the figures, the raw tomato extract is understood to effectively inhibit the expression of IL-6 mRNA in the results after three hours and after six hours.

### Experimental Example 4: effect of raw tomato extract on TNF-α mRNA expression in mouse microglial cells (MG6) in presence of LPS

For the purpose of comparison, the low-molecular-weight fraction (molecular weight: 10,000 or lower) as a raw tomato extract was analyzed for the effect of inhibiting the production of inflammatory cytokines.

The analysis was carried out by the same procedure as in Experimental Example 1, but using 100 ug/mL of low-molecular-weight raw tomato extract.

The results are shown in Fig. 7.

As demonstrated from the results shown in Fig. 7, the low-molecular-weight raw tomato extract is found to have no effect in inhibiting the production of inflammatory cytokines, while the high-molecular-weight fraction raw tomato extract having a molecular weight of 10,000 or higher is found to have an effect of inhibiting the production of inflammatory cytokines.

### Experimental Example 5: effect of raw tomato extract on TNF-α mRNA expression in mouse macrophage (Raw264) in presence of LPS

The analysis was carried out by the same procedure as in Experimental Example 1, but using a mouse macrophage cell line (Raw264) instead of the mouse microglial cell line (MG6).

Specifically, the analysis was carried out by the following specific procedure.
(1) A 6-well plate was seeded with a mouse macrophage cell line (Raw264) at 1 × 10⁵ cells/mL.
(2) After three to four days, solutions of four groups, i.e., control (DMEN medium, 1 mL/well), LPS (5 ng/mL), LPS (5 ng) + high-molecular-weight raw tomato extract (100 ug) (liquid volume: 1 mL), and high-molecular-weight raw tomato extract (100 ug/mL) were applied to the cells.
(3) After three hours and after six hours, the culture medium was removed and 0.3 mL of an RLT solution (RNeasy Mini kit, QIAGEN) (containing 1% β-mercaptoethanol) was added to each well.
(4) The cells were scraped using a cell scraper.
(5) The cell suspension was mixed with 0.3 mL of 70% ethanol.
(6) The above solution was put into a spin column (RNeasy Mini kit, QIAGEN) and centrifuged at 8000 × g for one minute.
(7) To the spin column was added 700 µL of an RW1 solution (RNeasy Mini kit, QIAGEN), followed by centrifugation at 8000 × g for one minute.
(8) To the spin column was added 500 µL of an RPE solution (RNeasy Mini kit, QIAGEN), followed by centrifugation at 8000 × g for one minute. This step was repeated twice.
(9) The spin column was placed in a new tube (1.5 mL) and 50 µL of RNase-free water was added. The mixture was left to stand for one minute, and then centrifuged at 12000 × g for one minute. This step was repeated twice.
(10) A 11 µL portion of this aqueous solution was used with Versco cDNA Synthesis Kit (Thermoscientific) to prepare cDNA.
(11) The resulting cDNA solution was diluted 10-fold with RNase-free water.
(12) This diluted cDNA solution was used to determine the expression of GAPDH mRNA and TNF-α mRNA by standard real-time RT-PCR.
(13) The PCR was performed using a 96-well plate.
(14) Each well was charged with 8.5 µL of water, 2.5 µL of cDNA sample solution, 1.5 µL of GAPDH mRNA detection primers or TNF-α mRNA detection primers (forward + reverse), and 12.5 µL of Syber green mater mix.
(15) The thermal cycle conditions for PCR were as follows: Hold, 94°C, 10 min + 3 step PCR (40 cycles; 94°C, 30 min + 55°C, 30 sec + 72°C, 30 sec) + Hold, 72°C, 1 min.
(16) For each sample of each group, the TNF-α mRNA/GAPDH mRNA value was determined and converted into percentage with the average value of the control taken as 100%.
(17) A statistical significance test was performed by analysis of variance followed by the Tukey-Kramer method.

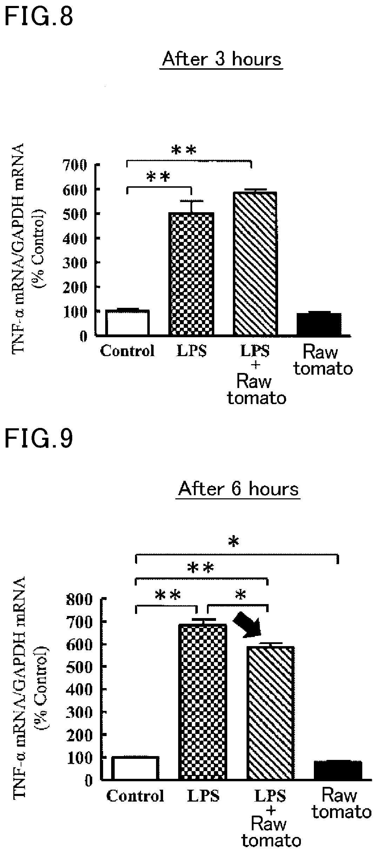

The results are shown in Figs. 8 and 9.

Fig. 8 shows the results after three hours and Fig. 9 shows the results after six hours.

As demonstrated from the results shown in Fig. 9, the raw tomato extract is understood to effectively inhibit the expression of TNF-α mRNA in the results after six hours.

### Experimental Example 6: effect of raw tomato extract on IL-1β mRNA expression in mouse macrophage (Raw264) in presence of LPS

The analysis was performed in accordance with the procedure of Experimental Example 5.

Specifically,
(1) A 6-well plate was seeded with a mouse macrophage cell line (Raw264) at 1 × 10⁵ cells/mL.
(2) After three to four days, solutions of four groups, i.e., control (DMEN medium, 1 mL/well), LPS (5 ng/mL), LPS (5 ng) + high-molecular-weight raw tomato extract (100 ug) (liquid volume: 1 mL), and high-molecular-weight raw tomato extract (100 ug/mL) were applied to the cells.
(3) After three hours and after six hours, the culture medium was removed and 0.3 mL of an RLT solution (RNeasy Mini kit, QIAGEN) (containing 1% β-mercaptoethanol) was added to each well.
(4) The cells were scraped using a cell scraper.
(5) The cell suspension was mixed with 0.3 mL of 70% ethanol.
(6) The above solution was put into a spin column (RNeasy Mini kit, QIAGEN) and centrifuged at 8000 × g for one minute.
(7) To the spin column was added 700 µL of an RW1 solution (RNeasy Mini kit, QIAGEN), followed by centrifugation at 8000 × g for one minute.
(8) To the spin column was added 500 µL of an RPE solution (RNeasy Mini kit, QIAGEN), followed by centrifugation at 8000 × g for one minute. This step was repeated twice.
(9) The spin column was placed in a new tube (1.5 mL) and 50 uL of RNase-free water was added. The mixture was left to stand for one minute, and then centrifuged at 12000 × g for one minute. This step was repeated twice.
(10) A 11-gL portion of this aqueous solution was used with Versco cDNA Synthesis Kit (Thermoscientific) to prepare cDNA.
(11) The resulting cDNA solution was diluted 10-fold with RNase-free water.
(12) This diluted cDNA solution was used to determine the expression of GAPDH mRNA and IL-1β mRNA by standard real-time RT-PCR.
(13) The PCR was performed using a 96-well plate.
(14) Each well was charged with 8.5 µL of water, 2.5 µL of cDNA sample solution, 1.5 µL of GAPDH mRNA detection primers or IL-1β mRNA detection primers (forward + reverse), and 12.5 µL of Syber green mater mix.
(15) The thermal cycle conditions for PCR were as follows: Hold, 94°C, 10 min + 3 step PCR (40 cycles; 94°C, 30 min + 55°C, 30 sec + 72°C, 30 sec) + Hold, 72°C, 1 min.
(16) For each sample of each group, the IL-1β mRNA/GAPDH mRNA value was determined and converted into percentage with the average value of the control taken as 100%.
(17) A statistical significance test was performed by analysis of variance followed by the Tukey-Kramer method.

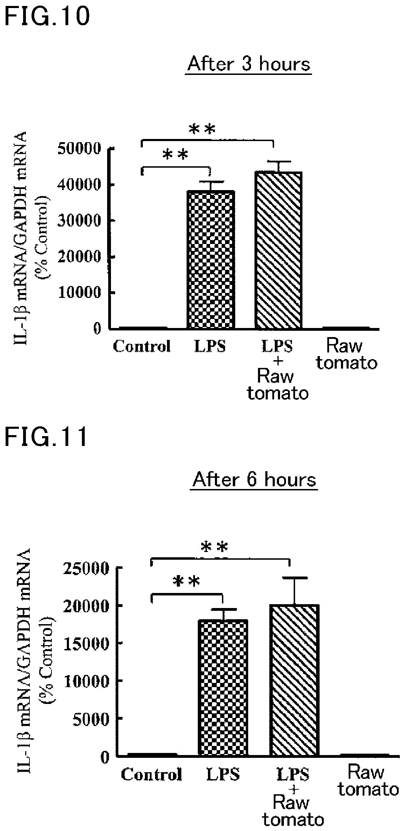

The results are shown in Figs. 10 and 11.

Fig. 10 shows the results after three hours and Fig. 11 shows the results after six hours.

### Experimental Example 7: effect of raw tomato extract on IL-6 mRNA expression in mouse macrophage (Raw264) in presence of LPS

The effect on IL-6 mRNA expression was analyzed using a mouse macrophage cell line (Raw264) in accordance with Experimental Examples 5 and 6.

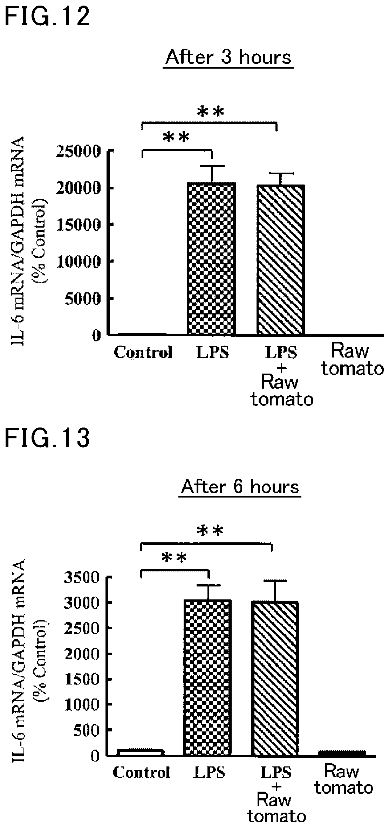

The results are shown in Figs. 12 and 13.

Fig. 12 shows the results after three hours and Fig. 13 shows the results after six hours.

From the above analysis results in Experimental Examples 5 to 7, the raw tomato extract is understood to significantly inhibit an increase in TNF-α mRNA expression caused by LSP stimulation, particularly in the mouse macrophage cell line (Raw264).

As can be well understood from the results of the above experimental examples, the raw tomato extract, which is a high-molecular-weight fraction having a molecular weight of 10,000 or higher, better exhibits an effect of inhibiting the production of inflammatory cytokines than the low-molecular-weight fraction. Therefore, it is demonstrated that the high-molecular-weight tomato extract in the present invention has an anti-inflammatory effect.

### INDUSTRIAL APPLICABILITY

The present invention provides inflammatory cytokine production inhibitors which are highly safe via oral administration or ingestion.

The inflammatory cytokine production inhibitors provided by the present invention inhibit overproduction of inflammatory cytokines such as interleukin-1 (IL-1), interleukin-6 (IL-6), and tumor necrosis factor (TNF-α), and thus may serve as therapeutic agents effective against a variety of diseases caused by overproduction of these cytokines, such as rheumatoid arthritis, ulcerative colitis, Crohn's disease, type 2 diabetes, obesity (especially insulin resistance), and depression. Further, these inhibitors have the advantage of providing daily healthcare effectively by daily oral ingestion. Accordingly, these inhibitors have significant industrial applicability.

## Claims

1. An inhibitor of the production of interleukin-1 (IL-1), interleukin-6 (IL-6), or tumor necrosis factor (TNF-α), which is a tomato extract fraction having a molecular weight of 10,000 or higher obtained by molecular weight fractionation of squeezed juice of raw tomatoes and from an extraction carried out using a centrifugal ultrafilter to obtain the extract as a fraction having a molecular weight of 10,000 or higher, for use as a medicament.

2. The inhibitor for use according to claim 1, wherein said inhibitor is in an orally administrable or orally ingestible form.

3. The inhibitor for use according to claim 1, wherein the use is a use in the treatment of inflammatory diseases caused by overproduction of these inflammatory cytokines, wherein the inflammatory disease is selected from rheumatoid arthritis, ulcerative colitis, Crohn's disease, and type 2 diabetes, depression, and obesity.

4. The inhibitor for use according to claims 1 to 3, wherein in said use, the inhibitor is administered daily by oral ingestion.

## Patentansprüche

1. Inhibitor der Produktion von Interleukin-1 (IL-1), Interleukin-6 (IL-6) oder Tumornekrosefaktor (TNF-α), der eine Fraktion von Tomatenextrakt mit einem Molekulargewicht von 10.000 oder mehr ist, die durch Molekulargewicht-Fraktionierung von gepresstem Saft von Rohtomaten und von einer Extraktion erhalten wird, die unter Verwendung eines Zentrifugal-Ultrafilters erhalten wird, um das Extrakt als eine Fraktion mit einem Molekulargewicht von 10.000 oder mehr zu erhalten, zur Verwendung als ein Medikament.

2. Inhibitor zur Verwendung nach Anspruch 1, wobei der Inhibitor in einer oral verabreichbaren oder oral aufnehmbaren Form vorliegt.

3. Inhibitor zur Verwendung nach Anspruch 1, wobei die Verwendung eine Verwendung bei der Behandlung von inflammatorischen Erkrankungen ist, die durch Überproduktion dieser inflammatorischen Cytokine verursacht wird, wobei die inflammatorische Erkrankung aus rheumatoider Arthritis, Colitis ulcerosa, Morbus Crohn und Typ-2-Diabetes, Depression und Fettsucht ausgewählt ist.

4. Inhibitor zur Verwendung nach Anspruch 1 bis 3, wobei der Inhibitor bei der Verwendung täglich durch orale Aufnahme verabreicht wird.

## Revendications

1. Inhibiteur de la production d'interleukine-1 (IL-1), d'interleukine-6 (IL-6), ou de facteur nécrosant les tumeurs (TNF-α), qui est une fraction d'extrait de tomate ayant une masse moléculaire de 10 000 ou plus, obtenue par fractionnement des masses moléculaires de jus pressé de tomates crues et à partir d'une extraction mise en oeuvre par utilisation d'un ultrafiltre centrifuge pour que soit obtenu l'extrait sous la forme d'une fraction ayant une masse moléculaire de 10 000 ou plus, pour une utilisation en tant que médicament.

2. Inhibiteur pour une utilisation selon la revendication 1, dans lequel ledit inhibiteur est sous une forme administrable par voie orale ou ingérable par voie orale.

3. Inhibiteur pour une utilisation selon la revendication 1, dans lequel l'utilisation est une utilisation dans le traitement de maladies inflammatoires causées par une surproduction de ces cytokines inflammatoires, dans lequel la maladie inflammatoire est choisie parmi la polyarthrite rhumatoïde, la recto-colite hémorragique, la maladie de Crohn, et le diabète de type 2, la dépression, et l'obésité.

4. Inhibiteur pour une utilisation selon les revendications 1 à 3, dans lequel, dans ladite utilisation, l'inhibiteur est administré quotidiennement par ingestion orale.
